# EUROPEAN PATENT SPECIFICATION

(11) **EP 2 262 493 B1**
(45) Date of publication and mention of the grant of the patent: **25.02.2015**
(21) Application number: 09717048.4
(22) Date of filing: 06.03.2009
(51) Int. Cl.: A61K 31/18, A61K 47/18, A61P 35/00

(54) **METHODS OF TREATMENT EMPLOYING PROLONGED CONTINUOUS INFUSION OF BELINOSTAT**
BEHANDLUNGSVERFAHREN MIT LÄNGERER KONTINUIERLICHER INFUSION VON BELINOSTAT
PROCÉDÉS DE TRAITEMENT EMPLOYANT UNE PERFUSION CONTINUE PROLONGÉE DE BELINOSTAT

(30) Priority: 07.03.2008 US 34635 P
(43) Date of publication of application: 22.12.2010
(73) Proprietor: Topotarget A/S, 2100 Copenhagen (DK)
(72) Inventor: SEHESTED, Maxwell, 2100 Copenhagen (DK); JENSEN, Peter Buhl, 2100 Copenhagen (DK); NISSEN, Nis, 2100 Copenhagen (DK)
(74) Representative: Wytenburg, Wilhelmus Johannes
(86) International application number: PCT/IB2009/005044
(87) International publication number: WO 2009/109861

(56) References cited:
- WO-A-2006/120456
- STEELE NICOLA L ET AL: "A phase 1 pharmacokinetic and pharmacodynamic study of the histone deacetylase inhibitor belinostat in patients with advanced solid tumors." CLINICAL CANCER RESEARCH : AN OFFICIAL JOURNAL OF THE AMERICAN ASSOCIATION FOR CANCER RESEARCH 1 FEB 2008, vol. 14, no. 3, 1 February 2008 (2008-02-01), pages 804-810, XP002531593 ISSN: 1078-0432
- GIMSING PETER: "Belinostat: a new broad acting antineoplastic histone deacetylase inhibitor." EXPERT OPINION ON INVESTIGATIONAL DRUGS APR 2009, vol. 18, no. 4, April 2009 (2009-04), pages 501-508, XP002531594 ISSN: 1744-7658
- GIMSING PETER ET AL: "A phase I clinical trial of the histone deacetylase inhibitor belinostat in patients with advanced hematological neoplasia." EUROPEAN JOURNAL OF HAEMATOLOGY SEP 2008, vol. 81, no. 3, September 2008 (2008-09), pages 170-176, XP002531595 ISSN: 1600-0609

## Description

### TECHNICAL FIELD

The present invention relates generally to the treatment of diseases and disorders that are mediated by histone deacetylase (HDAC), for example, cancer, with Belinostat, and more particularly, to improvement treatments of such diseases (for example, cancers, for example, leukemias), which employ prolonged continuous infusion (e.g., prolonged continuous intravenous infusion) of Belinostat.

### BACKGROUND

A number of patents and publications are cited herein in order to more fully describe and disclose the invention and the state of the art to which the invention pertains.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Thus, for example, reference to "a pharmaceutical carrier" includes mixtures of two or more such carriers, and the like.

Ranges are often expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

This disclosure includes information that may be useful in understanding the present invention. It is not an admission that any of the information provided herein is prior art or relevant to the presently claimed invention, or that any publication specifically or implicitly referenced is prior art.

### PXD101 / Belinostat

(E)-N-hydroxy-3-(3-phenylsulfamoyl-phenyl)-acrylamide, also known as PXD101, PX 105684, and Belinostat, shown below, is a well known histone deacetylate (HDAC) inhibitor. It was first described in Watkins et al., 2002. It is being developed for treatment of a range of disorders mediated by HDAC, and is the subject of a number of Phase I and Phase II trials for various cancers.

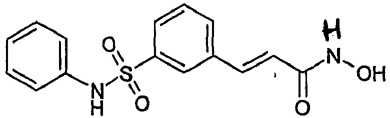

Liquid formulations of Belinostat, further comprising L-arginine, wherein the Belinostat is freely soluble, and which are suitable for administration by injection, infusion, intravenous infusion, etc., are described in Bastin et al., 2006.

Phase I dose finding studies have been performed in patients with various solid-tumours where 150 to 1200 mg/m² were given in an intravenous bolus over 30 minutes, giving a maximum tolerated dose of 1000 mg/m². See, e.g., Steele et al., 2008.

A 30 minute intravenous bolus of Belinostat (600-1200mg/m²/d) was also given to patients in combination with standard dose carboplatin or paclitaxel, where the maximum tolerated dose of Belinostat was 1000 mg/m²/d. See, e.g., Sinha et al., 2007.

Belinostat was also given to patients in a 30 minute intraveous bolus of 600-900 mg/m²/d. See, e.g., Gimsing et al., 2005.

Patients with multiple myeloma have been given 900-100 mg/m²/d Belinostat by 30 minute infusion. See, e.g., Sullivan et al., 2006.

Belinostat has been given at doses of 900 and 1000 mg/m²/d to patients with T-cell lymphoma. See, e.g., Advani et al., 2007.

Patients with drug-resistant ovarian tumours were given Belinostat at 1000 mg/m²/d in a 30 minutes intravenous bolus. See, e.g., Mackay et al., 2007.

Thus, in Phase I and II clinical trials, it has been reported that the recommended doses of Belinostat are given in as a bolus by 30 minute infusion on consecutive days. However, the reported plasma half-life of Belinostat is reported to be 47-86 minutes, and so the drug may not be at high enough concentrations to be effective for much of the treatment time.

Consequently, there is a need for an improved method of administration of Belinostat that would be more effective, as compared with the bolus doses previously described. There is also a need for an improved method of administration of Belinostat that would lead to increased efficacy while not exceeding dose-limiting toxicities.

### SUMMARY OF THE INVENTION

A first aspect of the invention is (E)-N-Hydroxy-3-(3-phenylsulfamoyl-phenyl)-acrylamide, or a salt, hydrate, or solvate thereof, for use in the treatment of a disease or disorder in a human patient, by prolonged continuous intravenous infusion;
wherein the prolonged continuous intravenous infusion is for a period of at least 12 hours;
wherein the disease or disorder is:
a proliferative condition; or
a tumour; or
a solid tumour; or
cancer; or
solid tumour cancer; or
lung cancer, prostate cancer, renal cancer, hepatoma, bladder cancer, colorectal cancer, pancreatic cancer, gastric cancer, breast cancer, ovarian cancer, soft tissue sarcoma, osteosarcoma, hepatocellular carcinoma, skin cancer, leukemia, or lymphoma; or
leukemia; or
acute myelogenous leukemia (AML), chronic myelogenous leukemia (CML), chronic myelogenous leukemia in blastic phase (CML-BP), or refractory myelodysplastic syndrome (MDS).

A second aspect of the invention is use of (E)-N-hydroxy-3-(3-phenylsulfamoyl-phenyl)-acrylamide, or a salt, hydrate, or solvate thereof, in the manufacture of a medicament for the treatment of a disease or disorder in a human patient, by prolonged continuous intravenous infusion;
wherein the prolonged continuous intravenous infusion is for a period of at least 12 hours;
wherein the disease or disorder is:
a proliferative condition; or
a tumour; or
a solid tumour; or
cancer; or
solid tumour cancer; or
lung cancer, prostate cancer, renal cancer, hepatoma, bladder cancer, colorectal cancer, pancreatic cancer, gastric cancer, breast cancer, ovarian cancer, soft tissue sarcoma, osteosarcoma, hepatocellular carcinoma, skin cancer, leukemia, or lymphoma; or
leukemia; or
acute myelogenous leukemia (AML), chronic myelogenous leukemia (CML), chronic myelogenous leukemia in blastic phase (CML-BP), or refractory myelodysplastic syndrome (MDS).

In one embodiment, the prolonged continuous intravenous infusion is for a period of at least 24 hours.

In one embodiment, the prolonged continuous intravenous infusion is for a period of at least 36 hours.

In one embodiment, the prolonged continuous intravenous infusion is for a period of at least 48 hours.

In one embodiment, the prolonged continuous intravenous infusion is performed for two or more cycles, with intervening rest periods.

In one embodiment, the rest period or each rest period is at least 24 hours.

In one embodiment, the rest period or each rest period is at least 6 days.

In one embodiment, the dosage during the or each prolonged continuous intravenous infusion is from 100 to 2500 mg/m²/d of (E)-N-hydroxy-3-(3-phenylsulfamoyl-phenyl)-acrylamide.
In one embodiment, the dosage during the or each prolonged continuous intravenous infusion is from 500 to 1500 mg/m²/d of (E)-N-hydroxy-3-(3-phenylsulfamoyl-phenyl)-acrylamide.

In one embodiment, the (E)-N-hydroxy-3-(3-phenylsulfamoyl-phenyl)-acrylamide is provided in a formulation suitable for administration by prolonged continuous intravenous infusion and further comprising L-arginine.

In one embodiment, the disease or disorder is a proliferative condition.

In one embodiment, the disease or disorder is cancer.

In one embodiment, the disease or disorder is leukemia

In one embodiment, the disease or disorder is acute myelogenous leukemia (AML), chronic myelogenous leukemia (CML), chronic myelogenous leukemia in blastic phase (CML-BP), or refractory myelodysplastic syndrome (MDS).

### BRIEF DESCRIPTION OF THE DRAWINGS

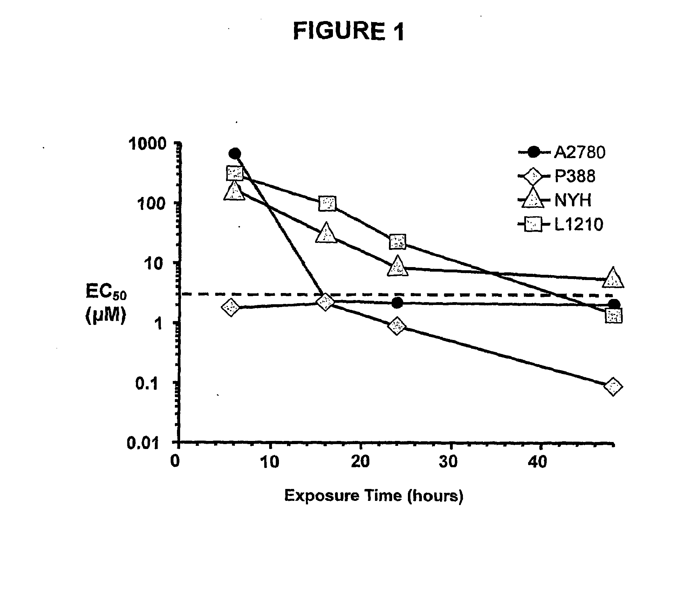

Figure 1 shows a graph of EC₅₀ (µM), as determined using a clonogenic assay as described herein, as a function of exposure time (hours) for four cells lines: P388 (diamonds), A2780 (circles), NYH (triangles), and L1210 (squares).

### DETAILED DESCRIPTION OF THE INVENTION

Described herein are methods of treatment of a patient suffering from a disease or disorder which is mediated by HDAC that involves the administration of a therapeutically-effective amount of Belinostat, or a salt, hydrate, or solvate thereof, to the patient by prolonged continuous infusion (e.g., prolonged continuous intravenous infusion).

Also described herein is a method of treatment of a disease or disorder which is mediated by HDAC in a patient, comprising administering a therapeutically-effective amount of Belinostat, or a salt, hydrate, or solvate thereof, to said patient by prolonged continuous infusion (e.g., prolonged continuous intravenous infusion).

Also described herein is Belinostat, or a salt, hydrate, or solvate thereof, for use in a method of treatment of a disease or disorder which is mediated by HDAC in a patient, by prolonged continuous infusion (e.g., prolonged continuous intravenous infusion).

Also described herein is use of Belinostat, or a salt, hydrate, or solvate thereof, in the manufacture of a medicament for the treatment of treatment of a disease or disorder which is mediated by HDAC in a patient, by prolonged continuous infusion (e.g., prolonged continuous intravenous infusion).

### Prolonged Continuous Infusion

In one embodiment, the prolonged continuous infusion (e.g., prolonged continuous intravenous infusion) is for a period of at least about 12 hours, for example, a period of from 12 to 24 hours, a period of from 12 to 48 hours, a period of from 12 to 72 hours, a period of from 12 to 96 hours, etc.

In one embodiment, the prolonged continuous infusion (e.g., prolonged continuous intravenous infusion) is for a period of at least about 16 hours, for example, a period of from 16 to 24 hours, a period of from 16 to 48 hours, a period of from 16 to 72 hours, a period of from 16 to 96 hours, etc.

In one embodiment, the prolonged continuous infusion (e.g., prolonged continuous intravenous infusion) is for a period of at least about 24 hours, for example, a period of from 24 to 48 hours, a period of from 24 to 72 hours, a period of from 24 to 96 hours etc.

In one embodiment, the prolonged continuous infusion (e.g., prolonged continuous intravenous infusion) is for a period of at least about 36 hours, for example, a period of from 36 to 48 hours, a period of from 36 to 72 hours, a period of from 36 to 96 hours etc.

In one embodiment, the prolonged continuous infusion (e.g., prolonged continuous intravenous infusion) is for a period of at least about 48 hours, for example, a period of from 48 to 72 hours, a period of from 48 to 96 hours etc.

In one embodiment, the prolonged continuous infusion (e.g., prolonged continuous intravenous infusion) is for a period of at least 72 hours, for example, a period of from 72 to 96 hours etc.

### Cycles of Administration

The prolonged continuous infusion (e.g., prolonged continuous intravenous infusion) may be performed one or more times (i.e., for one or more cycles), with intervening rest periods. Similarly, the prolonged continuous infusion (e.g., prolonged continuous intravenous infusion) may be performed two or more times (i.e., for two or more cycles), with intervening rest periods.

Each cycle may be the same or different. For example, if there are two cycles, they may, independently, have the same or different duration, the same or different dosage, etc.

In one embodiment, the prolonged continuous infusion (e.g., prolonged continuous intravenous infusion) is performed for two or more cycles, for example from 2 to 3 cycles, from 2 to 4 cycles, from 2 to 5 cycles, etc., with intervening rest periods.

In one embodiment, the prolonged continuous infusion (e.g., prolonged continuous intravenous infusion) is performed for three or more cycles, for example from 3 to 4 cycles, from 3 to 5 cycles, etc., with intervening rest periods.

In one embodiment, if the prolonged continuous infusion (e.g., prolonged continuous intravenous infusion) is performed for two or more cycles, then the rest period between cycles is at least about 12 hours, for example, from 12 to 24 hours, from 12 to 48 hours, from 12 hours to 3 days, from 12 hours to 6 days, from 12 hours to 13 days, from 12 hours to 20 days, etc.

In one embodiment, if the prolonged continuous infusion (e.g., prolonged continuous intravenous infusion) is performed for two or more cycles, then the rest period between cycles is at least about 24 hours, for example, from 24 to 48 hours, from 24 hours to 3 days, from 24 hours to 6 days, from 24 hours to 13 days, from 24 hours to 20 days, etc.

In one embodiment, if the prolonged continuous infusion (e.g., prolonged continuous intravenous infusion) is performed for two or more cycles, then the rest period between cycles is at least about 3 days, for example, from 3 to 6 days, from 3 to 13 days, from 3 to 20 days, etc.

In one embodiment, if the prolonged continuous infusion (e.g., prolonged continuous intravenous infusion) is performed for two or more cycles, then the rest period between cycles is at least about 6 days, for example, from 6 to 13 days, from 6 to 20 days, etc.

In one embodiment, if the prolonged continuous infusion (e.g., prolonged continuous intravenous infusion) is performed for two or more cycles, then the rest period between cycles is at least about 13 days, for example, from 13 to 20 days, etc.

### Route of Administration

In one embodiment, the administration is administration by infusion.
In one embodiment, the administration is administration by intravenous infusion.

"Infusion" differs from "injection" in that the term "infusion" describes the passive introduction of a substance (e.g., a fluid, electrolyte, etc.) into a vein or tissues by gravitational force, whereas the term "injection" describes the active introduction of a substance into a vein or tissues by additional forces, e.g., the pressure in a syringe. Intravenous infusion is often referred to as "intravenous drip" or "i.v. drip".

### Dosage

It will be appreciated by one of skill in the art that appropriate dosages of Belinostat (or a salt, hydrate, or solvate thereof), and compositions comprising Belinostat (or a salt, hydrate, or solvate thereof), can vary from patient to patient. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the patient. The amount of Belinostat (or a salt, hydrate, or solvate thereof) and route of administration will ultimately be at the discretion of the physician, veterinarian, or clinician, although generally the dosage will be selected to achieve local concentrations at the site of action which achieve the desired effect without causing substantial harmful or deleterious side-effects. In general, however, a suitable dose of Belinostat will be in the range of 100-2500 mg/m²/d, for example from 500-1500 mg/m²/d. Where the Belinostat is provided as a salt, hydrate, or solvate, the amount administered is calculated on the basis of the parent compound and so the actual weight to be used is increased proportionately.

In one embodiment, the dosage during the or each prolonged continuous infusion or the or each prolonged continuous intravenous infusion is from 100 to 2500 mg/m²/d of Belinostat.

In one embodiment, the dosage during the or each prolonged continuous infusion or the or each prolonged continuous intravenous infusion is from 500 to 1500 mg/m²/d of Belinostat.

### Belinostat

In one embodiment, the method employs Belinostat (also known (E)-N-hydroxy-3-(3-phenylsulfamoyl-phenyl)-acrylamide; PXD101; and PX 105684) or a salt, hydrate, or solvate thereof.

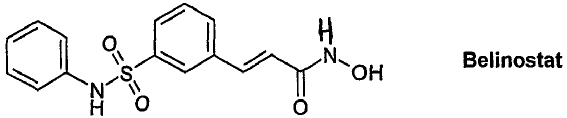

It may be convenient or desirable to prepare, purify, and/or handle a corresponding salt of Belinostat, for example, a pharmaceutically-acceptable salt. Examples of pharmaceutically acceptable salts are discussed in Berge et al., 1977, "Pharmaceutically Acceptable Salts," J. Pharm. Sci., Vol. 66, pp. 1-19.

Examples of suitable inorganic cations include, but are not limited to, alkali metal ions such as Na⁺ and K⁺, alkaline earth cations such as Ca²⁺ and Mg²⁺ , and other cations such as Al⁺³. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

It may be convenient or desirable to prepare, purify, and/or handle a corresponding solvate of Belinostat. The term "solvate" is used herein in the conventional sense to refer to a complex of solute (e.g., Belinostat, salt of Belinostat) and solvent. If the solvent is water, the solvate may be conveniently referred to as a hydrate, for example, a mono-hydrate, a di-hydrate, a tri-hydrate, etc.

In one preferred embodiment, the invention employs Belinostat.

### Conditions Treated

In one embodiment, the disease or disorder is a disease or disorder which is mediated by HDAC.

In one embodiment, the disease or disorder is a disease or disorder which is treatable or known to be treatable with an HDAC inhibitor.

In one embodiment, the disease or disorder is a proliferative condition.

In one embodiment, the disease or disorder is a tumour.

In one embodiment, the disease or disorder is a solid tumour.

In one embodiment, the disease or disorder is cancer.
In one embodiment, the disease or disorder is solid tumour cancer.

In one embodiment, the disease or disorder is lung cancer, prostate cancer, renal cancer, hepatoma, bladder cancer, colorectal cancer, pancreatic cancer, gastric cancer, breast cancer, ovarian cancer, soft tissue sarcoma, osteosarcoma, hepatocellular carcinoma, skin cancer, leukemia, or lymphoma.

In one embodiment, the disease or disorder is leukemia.

In one embodiment, the disease or disorder is acute myelogenous leukemia (AML), chronic myelogenous leukemia (CML), chronic myelogenous leukemia in blastic phase (CML-BP), or refractory myelodysplastic syndrome (MDS).

In one embodiment, the disease or disorder is acute myelogenous leukemia (AML).

In one embodiment, the disease or disorder is psoriasis.

In one embodiment, the disease or disorder is malaria.

### The Patient

In one embodiment, the patient is a mammal, i.e., a living mammal. In one embodiment, the patient is a human, i.e., a living human, including a living human foetus, a living human child, and a living human adult.

### Treatment

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, amelioration of the condition, and cure of the condition. Treatment as a prophylactic measure (i.e., prophylaxis) is also included. For example, use with subjects who have not yet developed the condition, but who are at risk of developing the condition, is encompassed by the term "treatment."

For example, treatment of a tumour may indicated by tumour reduction.

For leukemia, "tumour reduction" may be indicated by a reduction in blast cells (e.g., the number of blast cells, the percentage of blast cells) in the blood (e.g., peripheral blood) and/or the reduction of blast cells (e.g., the number of blast cells, the percentage of blast cells) in the bone marrow.

For solid tumours, "tumour reduction" may be indicated by a reduction of tumour mass, for example, as determined by radiographic examination (e.g., using PET and/or NMR methods) or by physical examination.

The term "therapeutically-effective amount," as used herein, pertains to that amount of Belinostat that is effective for producing some desired therapeutic effect, commensurate with a reasonable benefit/risk ratio, when administered in accordance with a desired treatment regimen.

The term "treatment" includes combination treatments and therapies, in which two or more treatments or therapies are combined, for example, sequentially or simultaneously. For example, Belinostat may also be used in combination therapies, e.g., in conjunction with other agents, for example, cytotoxic agents, etc. Examples of treatments and therapies include, but are not limited to, chemotherapy (the administration of active agents, including, e.g., HDAC inhibitors, antibodies (e.g., as in immunotherapy), prodrugs (e.g., as in photodynamic therapy, GDEPT, ADEPT, etc.); surgery; radiation therapy; and gene therapy.

### Formulations and Administration

As the methods relate to the administration of Belinostat (or a salt, hydrate, or solvate thereof) by prolonged continuous infusion (e.g., prolonged continuous intravenous infusion), the Belinostat (or a salt, hydrate, or solvate thereof) must be provided in a formulation suitable for parenteral administration, for example, a formulation suitable for administration by infusion, for example, a formulation suitable for administration by intravenous infusion. Guidance for suitable parenteral formulations is provided, for example, in Avis et al., 1992.

The Belinostat (or a salt, hydrate, or solvate thereof) is presented as a pharmaceutical formulation (e.g., composition, preparation, medicament) suitable for administration by infusion, and comprising Belinostat (or a salt, hydrate, or solvate thereof), together with one or more other pharmaceutically acceptable ingredients well known to those skilled in the art, including, but not limited to, pharmaceutically acceptable carriers, diluents, excipients, adjuvants, buffers, preservatives, anti-oxidants, stabilisers, solubilisers, surfactants (e.g., wetting agents), etc. The formulation may further comprise other active agents, for example, other therapeutic or prophylactic agents.

The term "pharmaceutically acceptable," as used herein, pertains to compounds, ingredients, materials, compositions, dosage forms, etc., which are, within the scope of sound medical judgment, suitable for use in contact with the tissues of the subject in question (e.g., mammal, human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation.

Suitable carriers, diluents, excipients, etc. can be found in standard pharmaceutical texts, for example, Remington's Pharmaceutical Sciences, 18th edition, Mack Publishing Company, Easton, Pa., 1990; and Handbook of Pharmaceutical Excipients, 5th edition, 2005.

The formulation may be prepared by any methods well known in the art of pharmacy.

The formulation may be prepared to provide for rapid or slow release; immediate, delayed, timed, or sustained release; or a combination thereof. The Belinostat, or a formulation comprising the Belinostat, may be presented in a liposome or other microparticulate which is designed to target the Belinostat, for example, to blood components or one or more organs.

The formulation may suitably be in the form of a liquid, a solution (e.g., aqueous, non-aqueous), a suspension (e.g., aqueous, non-aqueous), an emulsions (e.g., oil-in-water, water-in-oil), etc.

Formulations suitable for parenteral administration (e.g., by injection), include aqueous or non-aqueous, isotonic, pyrogen-free, sterile liquids (e.g., solutions, suspensions), in which the Belinostat is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

The preferred active ingredient, Belinostat, is sparingly soluble in water at physiological pH, and so must be administered in a pharmaceutical formulation where the Belinostat is freely soluble and the composition is well tolerated, for example, in combination with L-arginine, as described in Bastin et al., 2006.

In one embodiment, the Belinostat (or a salt, hydrate, or solvate thereof) is provided in a formulation suitable for parenteral administration and further comprising L-arginine, for example, a formulation suitable for administration by prolonged continuous infusion and further comprising L-arginine, for example, a formulation suitable for administration by prolonged continuous intravenous infusion and further comprising L-arginine.

Typically, parenteral formulations (i.e., formulations suitable for parenteral administration, e.g., intravenous infusion) are typically packaged in plastic or glass large volume parenteral (LVP) containers to which is attached a suitable intravenous (i.v.) set at the time of infusion. Venous entry is typically by a metal needle or plastic catheter.

A continuous infusion system provides continuous regulated fluid flow at a pre-set rate. Once a prescribed flow rate (e.g., 125 mL/hr) has been established, the fluid should continue to flow accurately from the system until the reservoir container has emptied.

The infusion may be infused according to a continuous or intermittent dose schedule. A continuous schedule typically involves the non-stop infusion of a relatively large volume of fluid (e.g., 1 litre per 8 hour period for adults). Continuous therapy typically additionally provides fluid, electrolytes, agents to adjust acid-base balance, nutrients, and some other drugs. The total fluid intake must not exceed the patient's requirements (approximately 2400 mL per day for an adult).

Accordingly, for use in the methods described herein, Belinostat (or a salt, hydrate, or solvate thereof) may be formulated for parenteral administration by prolonged continuous infusion, and may be presented, for example, in unit dose form in ampoules, pre-filled syringes, small volume infusion containers, or multi-dose containers optionally with an added preservative. The formulations may take such forms as suspensions, solutions or emulsions in oily or aqueous vehicles and may contain formulation agents such as suspending agents, stabilising agents, dispersing agents, etc.

### Kits

Also described herein is a kit comprising (a) Belinostat (or a salt, hydrate, or solvate thereof), or a composition comprising Belinostat (or a salt, hydrate, or solvate thereof), e.g., preferably provided in a suitable container and/or with suitable packaging; and (b) instructions for use, e.g., written instructions on how to administer the compound or composition as described herein, for example, by prolonged continuous infusion (e.g., prolonged continuous intravenous infusion).

The written instructions may also include a list of indications for which the active ingredient is a suitable treatment.

### EXAMPLES

The following examples are provided solely to illustrate the present invention.

### Study 1

### Clonogenic Assay of Cells with Increasing Time of Exposure to Belinostat

In order to determine Belinostat's concentration and exposure requirements for optimal efficacy, these two parameters were examined using the following cancer cell lines in vitro using a clonogenic assay: P388: mouse lymphocytic leukemia; A2780: human ovarian cancer; NYH: human small cell lung cancer; and L1210: mouse lymphocytic leukemia.

The method of the clonogenic assay is summarised in the following Table.

| Table 1 | |
|---|---|
| Clonogenic Assay Method | |
| 1. | 3.3% agar is boiled for at least 60 minutes in water bath on an electrical heating plate (30 mL PBS + 990 mg Bacto agar). |
| 2. | 90 mL growth medium (RPMI-1640 + 10% FCS) is heated on a water bath at 37°C. |
| 3. | Cells are centrifuged in 50 mL centrifuge tubes, at 1200 rpm for 5 minutes at room temperature. |
| 4. | Drug (Belinostat) is dissolved and diluted with growth medium or DMSO to give a concentration of x300 the intended final concentration. |
| 5. | Cells are suspended in 7 mL growth medium using a 1 mL syringe and an 18 gauge needle by pumping the solution up and down 15 times. |
| 6. | Cells are stained with Nigrosin (0.3 mL cells + 0.3 mL 0.1% Nigrosin in PBS), and counted after 8 minutes using a Fuchs-Rosenthal counting chamber, by counting 16 fields within the triple lines. Multiplying the count by 10,000 gives cells/mL. |
| 7. | The cells are diluted. Using 10,000 viable cells/mL for most cell lines will yield 2000 colonies in untreated controls, which is an appropriate cell concentration. |
| 8. | 10 mL agar and 90 mL growth medium is mixed (0.33%) and heated on a water bath at 37°C. |
| 9. | 0.35 mL cell suspension is transferred to 10 mL conical centrifuge tubes using a dispenser. 35 µL drug (Belinostat) is added. Five to seven different doses of drug, a non-treated control, and a vehicle control are made. |
| 10. | 3.15 mL agar/medium is added to each tube (maximum 8 tubes at a time). |
| 11. | Cells are seeded by seeding 1 mL of cell suspension in triplicate into 35 mm Petri dishes with sheep erythrocyte feeder layer after having re-suspended cells in each tube 6 + 4 times using a 1 mL syringe and a 18 gauge needle. |
| 12. | When the agar is solid (after about 1 hour), 1 mL of growth medium is carefully added to each dish using a pipette. The Petri dishes (18-24 dishes) are placed in ventilated 245 mm x 245 mm trays along with two Petri dishes with water. |
| 13. | Cells are counted after 14-21 days. |

The data are summarised in Figure 1, which is a graph of EC₅₀ (µM), as determined using the clonogenic assay described above, as a function of exposure time (hours) for the four cells lines, P388 (diamonds), A2780 (circles), NYH (triangles), and L1210 (squares).

As shown in Figure 1, Belinostat activity is both concentration and time dependent in all cell lines tested. Belinostat showed weak activity when incubation times were short, but the EC₅₀ values were markedly reduced for longer incubation (≥ 16 hours) with the drug.

### Study 2

### Tolerability of Belinostat in Dogs When Administered by Continuos Infusion

As long incubation times are required for the optimum efficacy of Belinostat in vitro, in vivo experiments were performed trying to mimic this situation by using prolonged (24 hour) continuous infusion in Beagle dogs. The purpose of this study was to determine a maximum tolerated dose for (a) Belinostat in L-arginine and (b) L-arginine alone, when administered via 24-hour continuous infusions (up to three times).

Belinostat, prepared in 4 mg/mL L-arginine in sterile water and formulated to give 0 mg/kg/hr, 0.5 mg/kg/hr, or 2 mg/kg/hr, was administered to groups of Beagle dogs via intravenous infusion at a rate of 1 mL/kg/hr for a number of continuous infusion periods, with intervening rest periods. Each group had one male and one female. The treatment schedule is summarised in the following Table.

| Table 2 | | | | | | | |
|---|---|---|---|---|---|---|---|
| Treatment Schedule for In Vivo Beagle Dog Study | | | | | | | |
| Grp. | Animal No./Sex | Treatment | SD | Date | ID (hr: min) | V_{T} (mL) | B_{T} (mg/ kg) |
| Phase A | | | | | | | |
| 1 | 12797M | L-Arginine (4 mg/kg/hr) | 1 | 09/08/05 | 24:00 | 271.4 | 0.0 |
| | 12798F | L-Arginine (4 mg/kg/hr) | 1 | 09/08/05 | 24:00 | 249.7 | 0.0 |
| 2 | 12799M | Belinostat (2 mg/kg/hr) + L-Arginine (4 mg/kg/hr) | 1 | 09/12/05 | 24:26 | 224.75 | 48.9 |
| | | | 2^{b} | 09/13/05 | 5:27 | 54.5 | 11.0 |
| | 12800F | Belinostat (2 mg/kg/hr) + L-Arginine (4 mg/kg/hr) | 1 | 09/12/05 | 24:22 | 219.35 | 48.7 |
| | | | 2^{b} | 09/13/05 | 5:01 | 59.45 | 12.0 |

| Phase B | | | | | | | |
|---|---|---|---|---|---|---|---|
| 3 | 12797M | L-Arginine (4 mg/kg/hr) | 1 | 09/28/05 | 23:58 | 294.85 | 0.0 |
| | | | 3^{c} | 09/30/05 | 24:00 | 276.0 | 0.0 |
| | | | 5^{c} | 10/02/05 | 24:00 | 276.0 | 0.0 |
| | 12798F | L-Arginine (4 mg/kg/hr) | 1 | 09/28/05 | 23:59 | 285.6 | 0.0 |
| | | | 3^{c} | 09/30/05 | 24:00 | 283.75 | 0.0 |
| | | | 5^{c} | 10/02/05 | 24:00 | 285.7 | 0.0 |
| | 12797M | Belinostat (2 mg/kg/hr) + L-Arginine (4 mg/kg/hr) | 9 | 10/06/05 | 24:00 | 283.2 | 48.0 |
| | 12798F | Belinostat (2 mg/kg/hr) + L-Arginine (4 mg/kg/hr) | 9 | 10/06/05 | 24:00 | 280.8 | 48.0 |

| Phase C | | | | | | | |
|---|---|---|---|---|---|---|---|
| 4 | 12885M | L-Arginine (1 mg/kg/hr) | 1 | 10/19/05 | 24:11 | 266.4 | 0.0 |
| | | | 3^{c} | 10/21/05 | 24:00 | 259.2 | 0.0 |
| | | | 5^{c} | 10/23/05 | 24:01 | 254.4 | 0.0 |
| | 12885F | L-Arginine (1 mg/kg/hr) | 1 | 10/19/05 | 24:00 | 273.6 | 0.0 |
| | | | 3^{c} | 10/21/05 | 24:00 | 249.6 | 0.0 |
| | | | 5^{c} | 10/23/05 | 24:00 | 244.8 | 0.0 |
| 5 | 12887M | Belinostat (0.5 mg/kg/hr) + L-Arginine (1 mg/kg/hr) | 1 | 10/19/05 | 24:00 | 244.8 | 12.0 |
| | | | 3^{c} | 10/21/05 | 24:00 | 266.4 | 12.0 |
| | | | 5^{c} | 10/23/05 | 24:00 | 261.6 | 12.0 |
| | 12888F | Belinostat (0.5 mg/kg/hr) + L-Arginine (1 mg/kg/hr) | 1 | 10/19/05 | 24:00 | 204.0 | 12.0 |
| | | | 3^{c} | 10/21/05 | 24:00 | 194.4 | 12.0 |
| | | | 5^{c} | 10/23/05 | 24:00 | 189.6 | 12.0 |

| | | | | | | | |
|---|---|---|---|---|---|---|---|
| SD - Study day. ID - Infusion duration (hr:min). V_{T} - Total volume received (mL). B_{T} - Total PXD101 received (mg/kg). (a) Study day indicates the day the infusion started. (b) Second infusion began almost immediately after the completion of the first infusion. (c) Infusion began approximately 24 hours after completion of previous infusion. Notes: (1) Animals were dosed at an infusion rate of 1 mL/kg/hr. (2) Animals 12797 and 12798 were dosed with 18 days of washout period between Phase A and B, and 2 days of washout period between Phase B and C. | | | | | | | |

Parameters evaluated during the study period included mortality, clinical, and cage-side observations, body weights, body temperature, gross pathology, and clinical pathology.

In addition, organ weight data were collected in Group 4 and 5 animals, and histopathology evaluation was performed on Group 3 animals.

Treatment with Belinostat at all dose levels, regardless of the L-arginine concentration, showed signs of severe toxicity.

The Group 2 animals (dosed with 2 mg/kg/hr Belinostat via 30-hour continuous infusion) were euthanized due to clinical signs (elevated body temperature, emesis, tremors, elevated heart rate, and body weight loss), and decreased white blood cells. Clinical chemistry results showed that aspartate aminotransferase (AST), creatine kinase (CK), cholesterol (CHOL), triglycerides (TRIG), glucose (GLU), and phosphorus (PHOS) were increased and calcium levels were decreased for these dogs.

The Group 3 animals (dosed with 2 mg/kg/hr Belinostat via 24-hour continuous infusion) were euthanized due to clinical signs (elevated body temperature, salivation, mucoid, soft, and/or discolored feces, and body weight losses). The microscopic examination of the animals suggested bone marrow hypoplasia/aplasia, widespread lymphoid depletion and necrosis, and epithelial necrosis in the gastrointestinal tract. In addition, the clinical hematology indicated decrease in myeloid and monocytic cell types in peripheral vasculature. Clinical chemistry results indicated increases in AST, CK, GLU, and CHOL and decreases in calcium (CA). PHOS levels increased in both dogs on Day 10 and fell on Day 13 for one dog.

The Group 5 animals (dosed with 0.5 mg/kg/hr PXD101 via three 24-hour continuous infusions with a 24-hour resting period between each infusion period) experienced clinical signs (mucoid and/or discolored faeces, emesis, hunched posture, body weight losses, and slightly increased body temperature). In addition, the clinical hematology indicated that the myeloid and monocytic cell types in peripheral vasculature, erythroid and lymphoid elements were also affected by treatment of Belinostat. GLU levels were elevated and CA levels were decreased for both dogs in this group.

Treatment with L-arginine alone did not produce any adverse effect. Sporadic observations of soft and/or mucoid faeces were observed, but were considered incidental because the observation was also noted predose. All other parameters were similar to pre-dose conditions.

Clinical signs (moribundity, emesis, discolored and/or soft feces), and hematological effects (decreased myeloid and monocytic cells types in peripheral vasculature) were evident at 0.5 mg/kg/hr Belinostat and higher. Additionally, bone marrow hypoplasia/aplasia, wide-spread lymphoid depletion and necrosis, and epithelial necrosis in the gastrointestinal tract were also observed at 2 mg/kg/hr Belinostat. Under the condition of the study, a maximum tolerated dose was not determined.

In summary, Belinostat was administered intravenously at 0, 0.5, 2 mg/kg/hr in 1 or 4 mg/kg/hr L-arginine for up to three 24-hour continuous infusion periods. Clinical signs (moribundity, emesis, discolored and/or soft faeces), and hematological effects (decreased myeloid and monocytic cells types in peripheral vasculature) were evident at dose levels .0.5 mg/kg/hr Belinostat. Additionally, bone marrow hypoplasia/aplasia, wide spread lymphoid depletion and necrosis, and epithelial necrosis in the gastrointestinal tract were also observed at 2 mg/kg/hr Belinostat. Therefore Belinostat is clearly highly toxic in dogs when given as a continuous infusion.

Note that the doses of Belinostat given to the dogs, 0.5 mg/kg/hr and 2 mg/kg/hr, correspond to 214 mg/m²/d and 857 mg/m²/d, respectively, which is of the same order as the dosages that have been given to humans by a 30 minutes bolus in clinical trials.

### Study 3

### Continuous Intravenous Infusion of Belinostat in a Human Subject

Despite the disappointing results for continuous infusion in dogs, Belinostat was given to a human patient by continuous infusion and, surprisingly and unexpectedly, was found to be well tolerated and efficacious.

The patient was a 71-year old woman with AML (acute myeloid leukemia). The patient had arterial hypertension since 1986, hypothyroidism since 1997 (treated with levothyroxin), anorexia, an enlarged spleen, night sweats since 2007, allergic exanthema since Jan 2008, and conjunctival hemorrhage from February 2007. AML was first diagnosed in December 2006. Prior treatment included four courses of decitabine from July 2007 to November 2007.

At presentation, and before treatment with Belinostat began, the patient had a high percentage of blasts in the bone marrow (75%), a high number of blasts in peripheral blood (2.4 x 10⁹/L), and correspondingly very few segment-neutrophils (0.3 x 10⁹/L).

The patient was to be treated with Belinostat (800 mg/m²/d) by continuous intravenous infusion for 48 hours, for a number of cycles, each 15 days apart (i.e., infusion on Days 1-2, 15-16, etc.).

The first infusion was tolerated well but had to be interrupted after 37 hours of infusion due to fever (39°C), dyspnea, and cough. Gram-negative infection was suspected (and likely, since the patient had very low neutrophil counts from the baseline) and antibiotics were provided. The patient recovered and was continued in the protocol with second cycle administered, as planned, on Day 15 and Day 16. A full 48 hour cycle was tolerated and no reports of serious adverse events have been received.

The following Table summarises the data for blasts, platelets, and white blood cells (WBC) for peripheral blood samples taken during the treatment.

| Table 3 | | | | | |
|---|---|---|---|---|---|
| Blast, Platelet, and White Blood Cell Levels | | | | | |
| Day | Date | Blasts (relative) (%) | Blasts (absolute) (x 10⁹/L) | Platelets (x 10⁹/L) | WBC (x 10⁹/L) |
| Pre-treatment | 04.02.08 | 65 | 2.4 | 133 | 3.8 |
| 24 h | 05.02.08 | 39 | 0.5 | 176 | 1.3 |
| 48 h | 06.02.08 | 28 | 0.2 | 138 | 0.6 |
| Day 5 | 08.02.08 | 55 | 1.6 | 80 | 2.8 |
| Day 8 | 11.02.08 | 48 | 2.0 | 39 | 4.2 |
| Day 15 (#) | 18.02.08 | 85 | 10.3 | 73 | 12.1 |
| Day 19 | 22.02.08 | 77 | 3.8 | 42 | 4.9 |
| Day 22 | 25.02.08 | 76 | 5.6 | 33 | 7.4 |
| Day 26 | 29.02.08 | 80 | 12.6 | 34 | 15.7 |

| | | | | | |
|---|---|---|---|---|---|
| (#) Before starting the 2nd cycle. | | | | | |

As demonstrated by the data, the absolute number of blasts in peripheral blood decreased during infusion from 2.4 x 10⁹/L immediately before starting treatment to 0.5 x 10⁹/L after 24 hours, and to 0.2 x 10⁹/L after 48 hours. The count then slowly recovered. The platelet count decreased from 133 x 10⁹/L to 39 x 10⁹/L after 24 hours, and to 28 x 10⁹/L after 48 hours.

Just before starting the second cycle on Day 15, the absolute number of blasts in peripheral blood had increased to 10.3 x 10⁹/L. In the second cycle, the absolute number of blasts in peripheral blood then decreased to 3.8 x 10⁹/L on Day 19 and 5.6 x 10⁹/L on Day 22. Platelet counts were moderately depressed also following second cycle.

Preliminary results from the clinical studies (like the one described above) show that prolonged exposures to Belinostat, as achieved by continuous infusion, are feasible, well tolerated and efficacious, despite animal studies suggesting that such scheduling results in unacceptable toxicities.

Continued enrolment on clinical trials will continue to define the recommended doses and infusion duration for Belinostat administered as a continuous infusion both in acute myeloid leukemia and in other cancers.

The foregoing has described the principles, preferred embodiments, and modes of operation of the present invention. The above-described embodiments should be regarded as illustrative rather than restrictive, and it should be appreciated that variations may be made in those embodiments by workers skilled in the art.

### REFERENCES

A number of patents and publications are cited above in order to more fully describe and disclose the invention and the state of the art to which the invention pertains. Full citations for these references are provided below.
Advani, R., et al., "Belinostat (PXD 101 in patients with recurrent or refractory peripheral or cutaneous T-cell lymphoma - results of a phase II study", 2007 Annual Meeting of the American Society for Haematology, 2007 ASH Annual Meeting Abstracts, Part 1, Volume 118, Issue 11, 16 November 2007, Abstract No 3453 (Poster Board 672, Session 111).
Avis, K.E. et al. (editors), 1992, "Pharmaceutical Dosage Forms: Parenteral Medications, Volume 1", second edition, pp. 514-518.
Bastin et al., 2006, "Pharmaceutical formulations of HDAC inhibitors", international patent application publication number WO/2006/120456 published 16 November 2007.
Gimsing et al., 2005, "Activity of the Histone Deacetylase (HDAC) Inhibitor PXD101 in Preclinical Studies and in a Phase I Study in Patients with Advanced Hematological Tumours", 2005 Annual Meeting of the American Society for Haematology, Blood (ASH Annual Meeting Abstracts), 2005, Vol. 106, Abstract No 3337.
Mackay, H.J., et al., 2007, "A phase II trial of the histone deacetylase inhibitor belinostat (PXD101) in patients with platinum resistant epithelial ovarian tumors and micropapillary/borderline (LMP) ovarian tumors. A trial of the PMH phase II Consortium," AACR-NCI-EORTC Annual Meeting 2007, American Association for Cancer Research: Molecular Targets and Cancer Therapeutics, October 22-26, 2007, San Francisco, CAA-160, 2007.
Sinha et al., 2007, "A phase I/II study of the safety and anticancer activity of iv-administered belinistat (PXD101) plus carboplatin or paclitaxel or both in patients with advanced solid tumours," 2007 Annual Meeting of the American Society of Clinical Oncology, Abstract No. 3574, page 156s, General Poster Session (Board #J7), Sunday 8;00AM-12:00PM.
Steele, N.L., et al, 2008, "A phase 1 pharmacokinetic and pharmacodynamic study of the histone deacetylase inhibitor Belinostat in patients with advanced solid tumors," Clin. Cancer Res., 01 February 2008, Vol. 14, No. 3, pp. 804-810.
Sullivan, D., et al., 2006, "A Phase II Study of PXD101 in Advanced Multiple Myeloma", 2006 Annual Meeting of the American Society for Haematology, 2006 ASH Annual Meeting Abstracts, Part 1, Volume 10B, Issue 11, 16 November 2007, page 1023a, Abstract No 3583 (Poster Board 812, Session 111).
Watkins et al., 2002, "Carbamic acid compounds comprising a sulfonamide linkage as HDAC inhibitors", international patent application publication number WO 02/30879 A2 published 18 April 2002.

## Claims

1. (E)-N-Hydroxy-3-(3-phenylsulfamoyl-phenyl)-acrylamide, or a salt, hydrate, or solvate thereof, for use in the treatment of a disease or disorder in a human patient, by prolonged continuous intravenous infusion;
wherein the prolonged continuous intravenous infusion is for a period of at least 12 hours;
wherein the disease or disorder is:
a proliferative condition; or
a tumour; or
a solid tumour; or
cancer; or
solid tumour cancer; or
lung cancer, prostate cancer, renal cancer, hepatoma, bladder cancer, colorectal cancer, pancreatic cancer, gastric cancer, breast cancer, ovarian cancer, soft tissue sarcoma, osteosarcoma, hepatocellular carcinoma, skin cancer, leukemia, or lymphoma; or
leukemia; or
acute myelogenous leukemia (AML), chronic myelogenous leukemia (CML), chronic myelogenous leukemia in blastic phase (CML-BP), or refractory myelodysplastic syndrome (MDS).

2. (E)-N-Hydroxy-3-(3-phenylsulfamoyl-phenyl)-acrylamide, or a salt, hydrate, or solvate thereof, for use according to claim 1, wherein the prolonged continuous intravenous infusion is for a period of at least 24 hours.

3. (E)-N-Hydroxy-3-(3-phenylsulfamoyl-phenyl)-acrylamide, or a salt, hydrate, or solvate thereof, for use according to claim 1, wherein the prolonged continuous intravenous infusion is for a period of at least 36 hours.

4. (E)-N-Hydroxy-3-(3-phenylsulfamoyl-phenyl)-acrylamide, or a salt, hydrate, or solvate thereof, for use according to claim 1, wherein the prolonged continuous intravenous infusion is for a period of at least 48 hours.

5. (E)-N-Hydroxy-3-(3-phenylsulfamoyl-phenyl)-acrylamide, or a salt, hydrate, or solvate thereof, for use according to any one of claims 1 to 4, wherein the prolonged continuous intravenous infusion is performed for two or more cycles, with intervening rest periods.

6. (E)-N-Hydroxy-3-(3-phenylsulfamoyl-phenyl)-acrylamide, or a salt, hydrate, or solvate thereof, for use according to claim 5, wherein the rest period or each rest period is at least 24 hours.

7. (E)-N-Hydroxy-3-(3-phenylsulfamoyl-phenyl)-acrylamide, or a salt, hydrate, or solvate thereof, for use according to claim 5, wherein the rest period or each rest period is at least 6 days.

8. (E)-N-Hydroxy-3-(3-phenylsulfamoyl-phenyl)-acrylamide, or a salt, hydrate, or solvate thereof, for use according to any one of claims 1 to 7, wherein the dosage during the or each prolonged continuous intravenous infusion is from 100 to 2500 mg/m²/d of (E)-N-hydroxy-3-(3-phenylsulfamoyl-phenyl)-acrylamide.

9. (E)-N-Hydroxy-3-(3-phenylsulfamoyl-phenyl)-acrylamide, or a salt, hydrate, or solvate thereof, for use according to any one of claims 1 to 7, wherein the dosage during the or each prolonged continuous intravenous infusion is from 500 to 1500 mg/m²/d of (E)-N-hydroxy-3-(3-phenylsulfamoyl-phenyl)-acrylamide.

10. (E)-N-Hydroxy-3-(3-phenylsulfamoyl-phenyl)-acrylamide, or a salt, hydrate, or solvate thereof, for use according to any one of claims 1 to 9, wherein the (E)-N-hydroxy-3-(3-phenylsulfamoyl-phenyl)-acrylamide is provided in a formulation suitable for administration by prolonged continuous intravenous infusion and further comprising L-arginine.

11. (E)-N-Hydroxy-3-(3-phenylsulfamoyl-phenyl)-acrylamide, or a salt, hydrate, or solvate thereof, for use according to any one of claims 1 to 10, wherein the disease or disorder is a proliferative condition.

12. (E)-N-Hydroxy-3-(3-phenylsulfamoyl-phenyl)-acrylamide, or a salt, hydrate, or solvate thereof, for use according to any one of claims 1 to 10, wherein the disease or disorder is cancer.

13. (E)-N-Hydroxy-3-(3-phenylsulfamoyl-phenyl)-acrylamide, or a salt, hydrate, or solvate thereof, for use according to any one of claims 1 to 10, wherein the disease or disorder is leukemia

14. (E)-N-Hydroxy-3-(3-phenylsulfamoyl-phenyl)-acrylamide, or a salt, hydrate, or solvate thereof, for use according to any one of claims 1 to 10, wherein the disease or disorder is acute myelogenous leukemia (AML), chronic myelogenous leukemia (CML), chronic myelogenous leukemia in blastic phase (CML-BP), or refractory myelodysplastic syndrome (MDS).

15. Use of (E)-N-hydroxy-3-(3-phenylsulfamoyl-phenyl)-acrylamide, or a salt, hydrate, or solvate thereof, in the manufacture of a medicament for the treatment of a disease or disorder in a human patient, by prolonged continuous intravenous infusion;
wherein the prolonged continuous intravenous infusion is for a period of at least12 hours;
wherein the disease or disorder is:
a proliferative condition; or
a tumour; or
a solid tumour; or
cancer; or
solid tumour cancer; or
lung cancer, prostate cancer, renal cancer, hepatoma, bladder cancer, colorectal cancer, pancreatic cancer, gastric cancer, breast cancer, ovarian cancer, soft tissue sarcoma, osteosarcoma, hepatocellular carcinoma, skin cancer, leukemia, or lymphoma; or
leukemia; or
acute myelogenous leukemia (AML), chronic myelogenous leukemia (CML), chronic myelogenous leukemia in blastic phase (CML-BP), or refractory myelodysplastic syndrome (MDS).

16. Use according to claim 15, wherein the prolonged continuous intravenous infusion is for a period of at least 24 hours.

17. Use according to claim 15, wherein the prolonged continuous intravenous infusion is for a period of at least 36 hours.

18. Use according to claim 15, wherein the prolonged continuous intravenous infusion is for a period of at least 48 hours.

19. Use according to any one of claims 15 to 18, wherein the prolonged continuous intravenous infusion is performed for two or more cycles, with intervening rest periods.

20. Use according to claim 19, wherein the rest period or each rest period is at least 24 hours.

21. Use according to claim 19, wherein the rest period or each rest period is at least 6 days.

22. Use according to any one of claims 15 to 21, wherein the dosage during the or each prolonged continuous intravenous infusion is from 100 to 2500 mg/m²/d of (E)-N-hydroxy-3-(3-phenylsulfamoyl-phenyl)-acrylamide.

23. Use according to any one of claims 15 to 21, wherein the dosage during the or each prolonged continuous intravenous infusion is from 500 to 1500 mg/m²/d of (E)-N-hydroxy-3-(3-phenylsulfamoyl-phenyl)-acrylamide.

24. Use according to any one of claims 15 to 23, wherein the (E)-N-hydroxy-3-(3-phenylsulfamoyl-phenyl)-acrylamide is provided in a formulation suitable for administration by prolonged continuous intravenous infusion and further comprising L-arginine.

25. Use according to any one of claims 15 to 24, wherein the disease or disorder is a proliferative condition.

26. Use according to any one of claims 15 to 24, wherein the disease or disorder is cancer.

27. Use according to any one of claims 15 to 24, wherein the disease or disorder is leukemia.

28. Use according to any one of claims 15 to 24, wherein the disease or disorder is acute myelogenous leukemia (AML), chronic myelogenous leukemia (CML), chronic myelogenous leukemia in blastic phase (CML-BP), or refractory myelodysplastic syndrome (MDS).

## Patentansprüche

1. (E)-N-Hydroxy-3-(3-phenylsulfamoylphenyl)acrylamid oder ein Salz, Hydrat oder Solvat davon zur Verwendung bei der Behandlung einer Krankheit oder Störung bei einem menschlichen Patienten durch kontinuierliche intravenöse Dauerinfusion;
worin die kontinuierliche intravenöse Dauerinfusion einen Zeitraum von zumindest 12 h in Anspruch nimmt;
worin es sich bei der Krankheit oder Störung um Folgendes handelt:
ein proliferatives Leiden; oder
einen Tumor; oder
einen soliden Tumor; oder
Krebs; oder
soliden Tumorkrebs; oder
Lungenkrebs, Prostatakrebs, Nierenkrebs, Hepatom, Blasenkrebs, Kolorektalkrebs, Bauchspeicheldrüsenkrebs, Magenkrebs, Brustkrebs, Eierstockkrebs, Weichgewebesarkom, Osteosarkom, hepatozelluläres Karzinom, Hautkrebs, Leukämie oder Lymphom; oder
Leukämie; oder
akute myelogene Leukämie (AML); chronische myelogene Leukämie (CML), chronische myelogene Leukämie in der Blastenphase (CML-BP) oder refraktäres myelodysplastisches Syndrom (MDS).

2. (E)-N-Hydroxy-3-(3-phenylsulfamoylphenyl)acrylamid oder ein Salz, Hydrat oder Solvat davon zur Verwendung nach Anspruch 1, worin die kontinuierliche intravenöse Dauerinfusion einen Zeitraum von zumindest 24 h in Anspruch nimmt.

3. (E)-N-Hydroxy-3-(3-phenylsulfamoylphenyl)acrylamid oder ein Salz, Hydrat oder Solvat davon zur Verwendung nach Anspruch 1, worin die kontinuierliche intravenöse Dauerinfusion einen Zeitraum von zumindest 36 h in Anspruch nimmt.

4. (E)-N-Hydroxy-3-(3-phenylsulfamoylphenyl)acrylamid oder ein Salz, Hydrat oder Solvat davon zur Verwendung nach Anspruch 1, worin die kontinuierliche intravenöse Dauerinfusion einen Zeitraum von zumindest 48 h in Anspruch nimmt.

5. (E)-N-Hydroxy-3-(3-phenylsulfamoylphenyl)acrylamid oder ein Salz, Hydrat oder Solvat davon zur Verwendung nach einem der Ansprüche 1 bis 4, worin die kontinuierliche intravenöse Dauerinfusion in zwei oder mehr Zyklen mit dazwischenliegenden Ruhephasen durchgeführt wird.

6. (E)-N-Hydroxy-3-(3-phenylsulfamoylphenyl)acrylamid oder ein Salz, Hydrat oder Solvat davon zur Verwendung nach Anspruch 5, worin die Ruhephase oder jede Ruhephase zumindest 24 h in Anspruch nimmt.

7. (E)-N-Hydroxy-3-(3-phenylsulfamoylphenyl)acrylamid oder ein Salz, Hydrat oder Solvat davon zur Verwendung nach Anspruch 5, worin die Ruhephase oder jede Ruhephase zumindest 6 Tage in Anspruch nimmt.

8. (E)-N-Hydroxy-3-(3-phenylsulfamoylphenyl)acrylamid oder ein Salz, Hydrat oder Solvat davon zur Verwendung nach einem der Ansprüche 1 bis 7, worin die Dosierung während der oder jeder kontinuierlichen intravenösen Dauerinfusion 100 bis 2500 mg/m²/d (E)-N-Hydroxy-3-(3-phenylsulfamoylphenyl)acrylamid beträgt.

9. (E)-N-Hydroxy-3-(3-phenylsulfamoylphenyl)acrylamid oder ein Salz, Hydrat oder Solvat davon zur Verwendung nach einem der Ansprüche 1 bis 7, worin die Dosierung während der oder jeder kontinuierlichen intravenösen Dauerinfusion 500 bis 1500 mg/m²/d (E)-N-Hydroxy-3-(3-phenylsulfamoylphenyl)acrylamid beträgt.

10. (E)-N-Hydroxy-3-(3-phenylsulfamoylphenyl)acrylamid oder ein Salz, Hydrat oder Solvat davon zur Verwendung nach einem der Ansprüche 1 bis 9, worin das (E)-N-Hydroxy-3-(3-phenylsulfamoylphenyl)acrylamid in einer zur Verabreichung mittels kontinuierlicher intravenöser Dauerinfusion geeigneten Formulierung bereitgestellt wird und des Weiteren L-Arginin umfasst.

11. (E)-N-Hydroxy-3-(3-phenylsulfamoylphenyl)acrylamid oder ein Salz, Hydrat oder Solvat davon zur Verwendung nach einem der Ansprüche 1 bis 10, worin die Krankheit oder Störung ein proliferatives Leiden ist.

12. (E)-N-Hydroxy-3-(3-phenylsulfamoylphenyl)acrylamid oder ein Salz, Hydrat oder Solvat davon zur Verwendung nach einem der Ansprüche 1 bis 10, worin die Krankheit oder Störung Krebs ist.

13. (E)-N-Hydroxy-3-(3-phenylsulfamoylphenyl)acrylamid oder ein Salz, Hydrat oder Solvat davon zur Verwendung nach einem der Ansprüche 1 bis 10, worin die Krankheit oder Störung Leukämie ist.

14. (E)-N-Hydroxy-3-(3-phenylsulfamoylphenyl)acrylamid oder ein Salz, Hydrat oder Solvat davon zur Verwendung nach einem der Ansprüche 1 bis 10, worin die Krankheit oder Störung akute myelogene Leukämie (AML); chronische myelogene Leukämie (CML), chronische myelogene Leukämie in der Blastenphase (CML-BP) oder refraktäres myelodysplastisches Syndrom (MDS) ist.

15. Verwendung von (E)-N-Hydroxy-3-(3-phenylsulfamoylphenyl)acrylamid oder einem Salz, Hydrat oder Solvat davon zur Herstellung eines Medikaments zur Behandlung einer Krankheit oder Störung bei einem menschlichen Patienten durch kontinuierliche intravenöse Dauerinfusion;
worin die kontinuierliche intravenöse Dauerinfusion einen Zeitraum von zumindest 12 h in Anspruch nimmt;
worin es sich bei der Krankheit oder Störung um Folgendes handelt:
eine proliferative Störung; oder
einen Tumor; oder
einen soliden Tumor; oder
Krebs; oder
soliden Tumorkrebs; oder
Lungenkrebs, Prostatakrebs, Nierenkrebs, Hepatom, Blasenkrebs, Kolorektalkrebs, Bauchspeicheldrüsenkrebs, Magenkrebs, Brustkrebs, Eierstockkrebs, Weichgewebesarkom, Osteosarkom, hepatozelluläres Karzinom, Hautkrebs, Leukämie oder Lymphom; oder
Leukämie; oder
akute myelogene Leukämie (AML); chronische myelogene Leukämie (CML), chronische myelogene Leukämie in der Blastenphase (CML-BP) oder refraktäres myelodysplastisches Syndrom (MDS).

16. Verwendung nach Anspruch 15, worin die kontinuierliche intravenöse Dauerinfusion einen Zeitraum von zumindest 24 h in Anspruch nimmt.

17. Verwendung nach Anspruch 15, worin die kontinuierliche intravenöse Dauerinfusion einen Zeitraum von zumindest 36 h in Anspruch nimmt.

18. Verwendung nach Anspruch 15, worin die kontinuierliche intravenöse Dauerinfusion einen Zeitraum von zumindest 48 h in Anspruch nimmt.

19. Verwendung nach einem der Ansprüche 15 bis 18, worin die kontinuierliche intravenöse Dauerinfusion in zwei oder mehr Zyklen mit dazwischenliegenden Ruhephasen durchgeführt wird.

20. Verwendung nach Anspruch 19, worin die Ruhephase oder jede Ruhephase zumindest 24 h in Anspruch nimmt.

21. Verwendung nach Anspruch 19, worin die Ruhephase oder jede Ruhephase zumindest 6 Tage in Anspruch nimmt.

22. Verwendung nach einem der Ansprüche 15 bis 21, worin die Dosierung während der oder jeder kontinuierlichen intravenösen Dauerinfusion 100 bis 2500 mg/m²/d (E)-N-Hydroxy-3-(3-phenylsulfamoylphenyl)acrylamid beträgt.

23. Verwendung nach einem der Ansprüche 15 bis 21, worin die Dosierung während der oder jeder kontinuierlichen intravenösen Dauerinfusion 500 bis 1500 mg/m²/d (E)-N-Hydroxy-3-(3-phenylsulfamoylphenyl)acrylamid beträgt.

24. Verwendung nach einem der Ansprüche 15 bis 23, worin das (E)-N-Hydroxy-3-(3-phenylsulfamoylphenyl)acrylamid in einer zur Verabreichung mittels kontinuierlicher intravenöser Dauerinfusion geeigneten Formulierung bereitgestellt wird und des Weiteren L-Arginin umfasst.

25. Verwendung nach einem der Ansprüche 15 bis 24, worin die Krankheit oder Störung ein proliferatives Leiden ist.

26. Verwendung nach einem der Ansprüche 15 bis 24, worin die Krankheit oder Störung Krebs ist.

27. Verwendung nach einem der Ansprüche 15 bis 24, worin die Krankheit oder Störung Leukämie ist.

28. Verwendung nach einem der Ansprüche 15 bis 24, worin die Krankheit oder Störung akute myelogene Leukämie (AML); chronische myelogene Leukämie (CML), chronische myelogene Leukämie in der Blastenphase (CML-BP) oder refraktäres myelodysplastisches Syndrom (MDS) ist.

## Revendications

1. (E)-N-hydroxy-3-(3-phénylsulfamoylphényl)acrylamide ou un sel, hydrate ou solvate de celui-ci, pour utilisation dans le traitement d'une maladie ou d'un trouble chez un patient humain, par perfusion intraveineuse continue prolongée ;
dans lequel la perfusion intraveineuse continue prolongée est effectuée sur une période d'au moins 12 heures ;
dans lequel la maladie ou le trouble est :
un état prolifératif ; ou
une tumeur ; ou
une tumeur solide ; ou
un cancer ; ou
un cancer à tumeur solide ; ou
un cancer pulmonaire, un cancer de la prostate, un cancer rénal, un hépatome, un cancer de la vessie, un cancer colorectal, un cancer pancréatique, un cancer gastrique, un cancer mammaire, un cancer ovarien, un sarcome de tissu mou, un ostéosarcome, un carcinome hépatocellulaire, un cancer de la peau, une leucémie, ou un lymphome ; ou
une leucémie ; ou
une leucémie myélogène aiguë (AML), une leucémie myélogène chronique (CML), une leucémie myélogène chronique en phase blastique (CML-BP), ou un syndrome myélodysplasique réfractaire (MDS).

2. (E)-N-hydroxy-3-(3-phénylsulfamoylphényl)acrylamide ou un sel, hydrate ou solvate de celui-ci, pour utilisation selon la revendication 1, dans lequel la perfusion intraveineuse continue prolongée est effectuée sur une période d'au moins 24 heures.

3. (E)-N-hydroxy-3-(3-phénylsulfamoylphényl)acrylamide ou un sel, hydrate ou solvate de celui-ci, pour utilisation selon la revendication 1, dans lequel la perfusion intraveineuse continue prolongée est effectuée sur une période d'au moins 36 heures.

4. (E)-N-hydroxy-3-(3-phénylsulfamoylphényl)acrylamide ou un sel, hydrate ou solvate de celui-ci, pour utilisation selon la revendication 1, dans lequel la perfusion intraveineuse continue prolongée est effectuée sur une période d'au moins 48 heures.

5. (E)-N-hydroxy-3-(3-phénylsulfamoylphényl)acrylamide ou un sel, hydrate ou solvate de celui-ci, pour utilisation selon l'une quelconque des revendications 1 à 4, dans lequel la perfusion intraveineuse continue prolongée est effectuée sur deux cycles ou plus, avec des périodes de repos intermédiaires.

6. (E)-N-hydroxy-3-(3-phénylsulfamoylphényl)acrylamide ou un sel, hydrate ou solvate de celui-ci, pour utilisation selon la revendication 5, dans lequel la période de repos ou chaque période de repos est d'au moins 24 heures.

7. (E)-N-hydroxy-3-(3-phénylsulfamoylphényl)acrylamide ou un sel, hydrate ou solvate de celui-ci, pour utilisation selon la revendication 5, dans lequel la période de repos ou chaque période de repos est d'au moins 6 jours.

8. (E)-N-hydroxy-3-(3-phénylsulfamoylphényl)acrylamide ou un sel, hydrate ou solvate de celui-ci, pour utilisation selon l'une quelconque des revendications 1 à 7, dans lequel la dose durant la ou chaque perfusion intraveineuse continue prolongée est de 100 à 2500 mg/m² par jour de (E)-N-hydroxy-3-(3-phénylsulfamoylphényl)acrylamide.

9. (E)-N-hydroxy-3-(3-phénylsulfamoylphényl)acrylamide ou un sel, hydrate ou solvate de celui-ci, pour utilisation selon l'une quelconque des revendications 1 à 7, dans lequel la dose durant la ou chaque perfusion intraveineuse continue prolongée est de 500 à 1500 mg/m² par jour de (E)-N-hydroxy-3-(3-phénylsulfamoylphényl)acrylamide.

10. (E)-N-hydroxy-3-(3-phénylsulfamoylphényl)acrylamide ou un sel, hydrate ou solvate de celui-ci, pour utilisation selon l'une quelconque des revendications 1 à 9, lequel (E)-N-hydroxy-3-(3-phénylsulfamoylphényl)acrylamide se présente sous une formulation adaptée pour une administration par perfusion intraveineuse continue prolongée, et comprend en outre de la L-arginine.

11. (E)-N-hydroxy-3-(3-phénylsulfamoylphényl)acrylamide ou un sel, hydrate ou solvate de celui-ci, pour utilisation selon l'une quelconque des revendications 1 à 10, dans lequel la maladie ou le trouble est un état prolifératif.

12. (E)-N-hydroxy-3-(3-phénylsulfamoylphényl)acrylamide ou un sel, hydrate ou solvate de celui-ci, pour utilisation selon l'une quelconque des revendications 1 à 10, dans lequel la maladie ou le trouble est un cancer.

13. (E)-N-hydroxy-3-(3-phénylsulfamoylphényl)acrylamide ou un sel, hydrate ou solvate de celui-ci, pour utilisation selon l'une quelconque des revendications 1 à 10, dans lequel la maladie ou le trouble est une leucémie.

14. (E)-N-hydroxy-3-(3-phénylsulfamoylphényl)acrylamide ou un sel, hydrate ou solvate de celui-ci, pour utilisation selon l'une quelconque des revendications 1 à 10, dans lequel la maladie ou le trouble est une leucémie myélogène aiguë (AML), une leucémie myélogène chronique (CML), une leucémie myélogène chronique en phase blastique (CML-BP), ou un syndrome myélodysplasique réfractaire (MDS).

15. Utilisation de (E)-N-hydroxy-3-(3-phénylsulfamoylphényl)acrylamide ou d'un sel, hydrate ou solvate de celui-ci, dans la fabrication d'un médicament destiné au traitement d'une maladie ou d'un trouble chez un patient humain, par perfusion intraveineuse continue prolongée ;
dans laquelle la perfusion intraveineuse continue prolongée est effectuée sur une période d'au moins 12 heures ;
dans laquelle la maladie ou le trouble est :
un état prolifératif ; ou
une tumeur ; ou
une tumeur solide ; ou
un cancer ; ou
un cancer à tumeur solide ; ou
un cancer pulmonaire, un cancer de la prostate, un cancer rénal, un hépatome, un cancer de la vessie, un cancer colorectal, un cancer pancréatique, un cancer gastrique, un cancer mammaire, un cancer ovarien, un sarcome de tissu mou, un ostéosarcome, un carcinome hépatocellulaire, un cancer de la peau, une leucémie, ou un lymphome ; ou
une leucémie ; ou
une leucémie myélogène aiguë (AML), une leucémie myélogène chronique (CML), une leucémie myélogène chronique en phase blastique (CML-BP), ou un syndrome myélodysplasique réfractaire (MDS).

16. Utilisation selon la revendication 15, dans laquelle la perfusion intraveineuse continue prolongée est effectuée sur une période d'au moins 24 heures.

17. Utilisation selon la revendication 15, dans laquelle la perfusion intraveineuse continue prolongée est effectuée sur une période d'au moins 36 heures.

18. Utilisation selon la revendication 15, dans laquelle la perfusion intraveineuse continue prolongée est effectuée sur une période d'au moins 48 heures.

19. Utilisation selon l'une quelconque des revendications 15 à 18, dans laquelle la perfusion intraveineuse continue prolongée est effectuée sur deux cycles ou plus, avec des périodes de repos intermédiaires.

20. Utilisation selon la revendication 19, dans laquelle la période de repos ou chaque période de repos est d'au moins 24 heures.

21. Utilisation selon la revendication 19, dans laquelle la période de repos ou chaque période de repos est d'au moins 6 jours.

22. Utilisation selon l'une quelconque des revendications 15 à 21, dans laquelle la dose durant la ou chaque perfusion intraveineuse continue prolongée est de 100 à 2500 mg/m² par jour de (E)-N-hydroxy-3-(3-phénylsulfamoylphényl)acrylamide.

23. Utilisation selon l'une quelconque des revendications 15 à 21, dans laquelle la dose durant la ou chaque perfusion intraveineuse continue prolongée est de 500 à 1500 mg/m² par jour de (E)-N-hydroxy-3-(3-phénylsulfamoylphényl)acrylamide.

24. Utilisation selon l'une quelconque des revendications 15 à 23, dans laquelle le (E)-N-hydroxy-3-(3-phénylsulfamoylphényl)acrylamide se présente sous une formulation adaptée pour une administration par perfusion intraveineuse continue prolongée, et comprend en outre de la L-arginine.

25. Utilisation selon l'une quelconque des revendications 15 à 24, dans laquelle la maladie ou le trouble est un état prolifératif.

26. Utilisation selon l'une quelconque des revendications 15 à 24, dans laquelle la maladie ou le trouble est un cancer.

27. Utilisation selon l'une quelconque des revendications 15 à 24, dans laquelle la maladie ou le trouble est une leucémie.

28. Utilisation selon l'une quelconque des revendications 15 à 24, dans laquelle la maladie ou le trouble est une leucémie myélogène aiguë (AML), une leucémie myélogène chronique (CML), une leucémie myélogène chronique en phase blastique (CML-BP), ou un syndrome myélodysplasique réfractaire (MDS).
